# EUROPEAN PATENT APPLICATION

(11) **EP 2 208 484 A1**
(43) Date of publication of application: **21.07.2010**
(21) Application number: 08848527.1
(22) Date of filing: 05.11.2008
(51) Int. Cl.: A61F 7/10

(54) **COOLING TOOL**

(30) Priority: 07.11.2007 JP 2007008616 U; 25.04.2008 JP 2008002685 U; 05.09.2008 JP 2008006299 U
(71) Applicant: Hirakawa Corporation, Chuo-ku Tokyo 103-0014 (JP)
(72) Inventor: HIRAKAWA, Shoichi, Tokyo 103-0014 (JP)
(74) Representative: Elzaburu Marquez, Alberto
(86) International application number: PCT/JP2008/070143
(87) International publication number: WO 2009/060876

(57) **Abstract**

Problem to be Solved: A third invention provides a human body cooling apparatus which suppresses uneven distribution due to the migratability of a built-in cooling medium, thereby exhibiting a sufficient cooling effect for the body. Solution: The human body cooling apparatus (301) pertaining to the third invention includes an exterior element (302) in which two covering members in the shape of a rectangular seat each having a front cloth formed of a material selected from cotton fiber, polyester, rayon, hemp, raised fabric, crepe, striped crepe, towel fabric, and the like, and a backing cloth formed of a non-water absorbent material, such as polyvinyl chloride, or the like, which is weldable by high-frequency heating are put one upon another to provide a flat form, said backing cloths being superposed on each other and the backing cloths being high-frequency welded for formation of an outer edge crimped portion (306), a toric partially crimped portion (307) having an air bleeder hole 308 for heat radiation in a plurality of places in an internal area thereof, and straight-linear middle crimped portions (309); and a cooling medium (305) containing gel, sodium polyacrylate, ammonium sulfate, sodium sulfate, glycerin, water, fungicide, and the like, accommodated in said exterior element (302) such that migration thereof is restricted by said outer edge crimped portion (306), partially crimped portions (307) in the internal area thereof, and middle crimped portions (309) said exterior element (302) being capable of reversible use by front/back reversing it.

## Description

### Technical Field

The present invention includes a first invention, a second invention, and a third invention, and the respective inventions relates to a human body cooling apparatus, and particularly, relates to a human body cooling apparatus which utilizes the cooling effect of a cooling medium containing gel which is used for cooling each portion of a human body.

### Background Art

Conventionally, people has accommodated a cooling medium containing gel in an exterior element of a pillow size or a bed sheet size to form a human body cooling apparatus, and at the time of sleeping, or the like, in the high temperature season, has used this human body cooling apparatus with a pillow for cooling the head, or placed it on the bed for cooling the back, and the like, of the body.

With such a cooling pad, the migratability of a cooling medium containing gel accommodated inside thereof is high, thus if the cooling medium is only accommodated in the exterior element, the weight of the body would cause the cooling medium to be unevenly distributed in the exterior element, resulting in a sufficient cooling effect for the body being not capable of being expected.

In addition, for such a cooling pad, it is also required to take the function of radiating the heat from the body into account.

In the patent document 1, a cooling/heating gel pillow including a cooling/heating gel sheet of a pillow size, and a pillow cover having an accommodation part inside thereof into/from which this cooling/heating gel sheet can be inserted/removed is disclosed.

However, it is a main object of the cooling/heating gel pillow as disclosed in the patent document 1 to provide the cooling effect thereof for the head of human body, and the size thereof is relatively small, thus in consideration of the migratability of the water-containing gel, it is estimated that, even if the configuration of such a cooling/heating gel pillow is applied, as it is, to a human body cooling apparatus of a long size, such as a bed sheet size, or the like, no sufficient cooling effect can be expected.
Patent document 1: Japanese Patent Laid-Open Publication No. 2002-23442

### Disclosure of the Invention

### Problems to be Solved by the Invention:

The problem to be solved by the first invention is a fact that, in any sizes ranging from a long size, such as a bed sheet size, to a relatively small size, such as a pillow size, no human body cooling apparatus is available which can suppress uneven distribution due to the migratability of the cooling medium and exhibit a sufficient cooling effect for the body.

The problem to be solved by the second invention is a fact that, in any sizes ranging from a long size, such as a bed sheet size, to a relatively small size, such as a pillow size, no human body cooling apparatus is available which can suppress uneven distribution due to the migratability of the cooling medium and exhibit a sufficient cooling effect for the body.

The problem to be solved by the third invention is a fact that, in any sizes ranging from a long size, such as a bed sheet size, to a relatively small size, such as a pillow size, no human body cooling apparatus is available which can suppress uneven distribution due to the migratability of the cooling medium and exhibit a sufficient cooling effects for the body, and is excellent in function of radiating the heat.

### Means for Solving the Problems:

The human body cooling apparatus of the first invention provides, as a most important feature thereof, a human body cooling apparatus, including an exterior element in which two covering members in the shape of a rectangular seat each having a front cloth made of fabric, and a backing cloth which is weldable by high-frequency heating are put one upon another to provide a flat form, said backing cloths being superposed on each other; and a cooling medium containing gel accommodated in said exterior element such that migration thereof is restricted by an outer edge crimped portion and a partially crimped portion in an internal area thereof that are produced by high-frequency welding of the backing cloths in said two covering members.

The human body cooling apparatus of the second invention provides, as a most important feature thereof, a human body cooling apparatus, including an exterior element in which two covering members in the shape of a rectangular seat each having a front cloth formed of a material selected from cotton fiber, polyester, rayon, hemp, raised fabric, crepe, striped crepe, towel fabric, and the like, and a backing cloth formed of a non-water absorbent material, such as polyvinyl chloride, or the like, which is weldable by high-frequency heating are put one upon another to provide a flat form, said backing cloths being superposed on each other; and a cooling medium containing gel, sodium polyacrylate, ammonium sulfate, sodium sulfate, glycerin, water, fungicide, and the like, accommodated in said exterior element such that migration thereof is restricted by an outer edge crimped portion and a partially crimped portion in an internal area thereof that are produced by high-frequency welding of the backing cloths in said two covering members.

The human body cooling apparatus of the third invention provides, as a most important feature thereof, a human body cooling apparatus, including an exterior element in which two covering members in the shape of a rectangular seat each having a front cloth formed of a fabric-like material, and a backing cloth formed of a non-water absorbent material which is weldable by high-frequency heating are put one upon another to provide a flat form, said backing cloths being superposed on each other, and the backing cloths being high-frequency welded for formation of an outer edge crimped portion, a toric partially crimped portion having an air bleeder hole for heat radiation in a plurality of places in an internal area thereof, and straight-linear middle crimped portions; and a cooling medium accommodated in said exterior element such that migration thereof is restricted by said outer edge crimped portion, partially crimped portions in the internal area thereof, and middle crimped portions.

### Advantages of the Invention:

According to the human body cooling apparatuses of the first invention as stated in the respective claims, human body cooling apparatuses which can exhibit the following effects can be provided.

The invention as stated in Claim 1 can provide a human body cooling apparatus in which migration of a cooling medium containing gel accommodated in an exterior element is restricted by an outer edge crimped portion and a partially crimped portion in an internal area thereof that are produced by high-frequency welding of the backing cloths in two covering members, whereby uneven distribution is eliminated, and at the time of sleeping, or the like, a sufficient cooling effect for the body can be exhibited.

The invention as stated in Claim 2 can provide a human body cooling apparatus which, having a size selected from various sizes such as a bed sheet size, a pillow size, a half size, i.e., a middle size between these sizes, further, a car seat size, a floor cushion size, a wine bottle heat insulating seat size, and the like, can exhibit a sufficient cooling effect for the entire body, the head, or a part of the body at the time of sleeping, or the like, in the same manner as with the invention as stated in Claim 1.

The invention as stated in Claim 3 can provide a human body cooling apparatus which, with a configuration having a four-side outer edge crimped portion and a toric partially crimped portion in a plurality of places in an internal area thereof that are produced by high-frequency welding of the backing cloths in two covering members, can exhibit the same cooling effect as that with the invention as stated in Claim 2.

The invention as stated in Claim 4 can provide a human body cooling apparatus which, with a configuration having a four-side outer edge crimped portion, a cross-shaped partially crimped portion connecting between the middle portions of the respective opposed sides in the four-side outer edge crimped portion, and a plurality of rectangular partially crimped portions in the respective four areas defined by the cross-shaped partially crimped portion that are produced by high-frequency welding of the backing cloths in two covering members, can exhibit the same cooling effect as that with the invention as stated in Claim 2.

The invention as stated in Claim 5 can provide a human body cooling apparatus which, with a configuration having a four-side outer edge crimped portion and a plurality of corrugated partially crimped portions in an internal area thereof that are produced by high-frequency welding of the backing cloths in two covering members, can exhibit the same cooling effect as that with the invention as stated in Claim 2.

The invention as stated in Claim 6 can provide a human body cooling apparatus which, with a configuration having a four-side outer edge crimped portion and a plurality of rectangular partially crimped portions in an internal area thereof that are produced by high-frequency welding of the backing cloths in two covering members, can exhibit the same cooling effect as that with the invention as stated in Claim 2.

The invention as stated in Claim 7 can provide a human body cooling apparatus which, with a configuration having a four-side outer edge crimped portion and a plurality of partially crimped portions arranged in parallel to one another in an inclined disposition in an internal area thereof to exhibit a substantially rectangular space occupying configuration as a whole that are produced by high-frequency welding of the backing cloths in said two covering members, can exhibit the same cooling effect as that with the invention as stated in Claim 2.

The invention as stated in Claim 8 can provide a human body cooling apparatus which, with a configuration having a four-side outer edge crimped portion and a plurality of partially crimped portions arranged in a pattern of crosses in an internal area thereof, each cross exhibiting a substantially rectangular space occupying configuration as a whole, that are formed by high-frequency welding of the backing cloths in two covering members, can exhibit the same cooling effect as that with the invention as stated in Claim 2.

According to the human body cooling apparatuses of the second invention as stated in the respective claims, human body cooling apparatuses which can exhibit the following effects can be provided.

The invention as stated in Claim 9 can provide a human body cooling apparatus in which migration of a cooling medium containing gel, sodium polyacrylate, ammonium sulfate, sodium sulfate, glycerin, water, fungicide, and the like, accommodated in an exterior element is restricted by an outer edge crimped portion and a partially crimped portion in an internal area thereof that are produced by high-frequency welding of the backing cloths in two covering members in the shape of a rectangular seat each having a front cloth formed of a material selected from cotton fiber, polyester, rayon, hemp, raised fabric, crepe, striped crepe, towel fabric, and the like, and a backing cloth formed of a non-water absorbent material, such as polyvinyl chloride, or the like, which is weldable by high-frequency heating, whereby uneven distribution is eliminated, and at the time of sleeping, or the like, a sufficient cooling effect for the body can be exhibited.

The invention as stated in Claim 10 can provide a human body cooling apparatus which, with substantially the same configuration as that of the invention as stated in Claim 9, and having a size selected from various sizes such as a bed sheet size, a pillow size, a half size, i.e., a middle size between these sizes, further, a car seat size, a floor cushion size, a wine bottle heat insulating seat size, and the like, can exhibit a sufficient cooling effect for the entire body, the head, or a part of the body at the time of sleeping, or the like, in the same manner as with the invention as stated in Claim 9, and is also capable of reversible use by front/back reversing of an exterior element.

The invention as stated in Claim 11 can provide a human body cooling apparatus which, with substantially the same configuration as that of the invention as stated in Claim 9, and a configuration having a four-side outer edge crimped portion and a toric partially crimped portion in a plurality of places in an internal area thereof that are produced by high-frequency welding of the backing cloths in two covering members, can exhibit the same cooling effect as that with the invention as stated in Claim 10, and is also capable of reversible use by front/back reversing of an exterior element.

The invention as stated in Claim 12 can provide a human body cooling apparatus which, with substantially the same configuration as that of the invention as stated in Claim 9, and a configuration having a four-side outer edge crimped portion, a cross-shaped partially crimped portion connecting between the middle portions of the respective opposed sides in the four-side outer edge crimped portion, and a plurality of rectangular partially crimped portions in the respective four areas defined by the cross-shaped partially crimped portion that are produced by high-frequency welding of the backing cloths in two covering members, can exhibit the same cooling effect as that with the invention as stated in Claim 10, and is also capable of reversible use by front/back reversing of an exterior element.

The invention as stated in Claim 13 can provide a human body cooling apparatus which, with substantially the same configuration as that of the invention as stated in Claim 9, and a configuration having a four-side outer edge crimped portion and a plurality of corrugated partially crimped portions in an internal area thereof that are produced by high-frequency welding of the backing cloths in two covering members, can exhibit the same cooling effect as that with the invention as stated in Claim 10, and is also capable of reversible use by front/back reversing of an exterior element.

The invention as stated in Claim 14 can provide a human body cooling apparatus which, with substantially the same configuration as that of the invention as stated in Claim 9, and a configuration having a four-side outer edge crimped portion and a plurality of rectangular partially crimped portions in an internal area thereof that are produced by high-frequency welding of the backing cloths in two covering members, can exhibit the same cooling effect as that with the invention as stated in Claim 10, and is also capable of reversible use by front/back reversing of an exterior element.

The invention as stated in Claim 15 can provide a human body cooling apparatus which, with substantially the same configuration as that of the invention as stated in Claim 9, and a configuration having a four-side outer edge crimped portion and a plurality of partially crimped portions arranged in a pattern of crosses in an internal area thereof, each cross exhibiting a substantially rectangular space occupying configuration as a whole, that are formed by high-frequency welding of the backing cloths in two covering members, can exhibit the same cooling effect as that with the invention as stated in Claim 10, and is also capable of reversible use by front/back reversing of an exterior element.

The invention as stated in Claim 16 can provide a human body cooling apparatus which, with substantially the same configuration as that of the invention as stated in Claim 9, and a configuration having a four-side outer edge crimped portion and a plurality of partially crimped portions arranged in a pattern of crosses in an internal area thereof, each cross exhibiting a substantially rectangular space occupying configuration as a whole, that are formed by high-frequency welding of the backing cloths in two covering members, can exhibit the same cooling effect as that with the invention as stated in Claim 10, and is also capable of reversible use by front/back reversing of said exterior element.

According to the human body cooling apparatuses of the third invention as stated in the respective claims, human body cooling apparatuses which can exhibit the following effects can be provided.

The invention as stated in Claim 17 can provide a human body cooling apparatus in which an exterior element is formed to a flat configuration by mutually superposing two covering members in the shape of a rectangular seat each having a front cloth formed of a fabric-like material, and a backing cloth formed of a non-water absorbent material which is weldable by high-frequency heating, with said backing cloths being mutually superposed and mutually high-frequency welded for formation of an outer edge crimped portion, a toric partially crimped portion having an air bleeder hole for heat radiation in a plurality of places in an internal area thereof, and straight-linear middle crimped portions; and a cooling medium is accommodated in said exterior element such that migration thereof is restricted by said outer edge crimped portion, partially crimped portions in the internal area thereof, and middle crimped portions, whereby migration of the cooling medium is restricted by said partially crimped portion and middle crimped portion, resulting in uneven distribution being eliminated, and by an air bleeder hole provided in said respective partially crimped portions, heat radiation is satisfactorily performed, which allows a sufficient cooling effect for the body to be exhibited at the time of sleeping, or the like.

The invention as stated in Claim 18 can provide a human body cooling apparatus in which an exterior element is formed to a flat configuration by mutually superposing two covering members in the shape of a rectangular seat each having a front cloth formed of a material selected from cotton fiber, polyester, rayon, hemp, raised fabric, crepe, striped crepe, towel fabric, and the like, and a backing cloth formed of a non-water absorbent material, such as polyvinyl chloride, or the like, which is weldable by high-frequency heating, and a backing cloth formed of a non-water absorbent material which is weldable by high-frequency heating, with said backing cloths being mutually superposed and mutually high-frequency welded for formation of an outer edge crimped portion, a toric partially crimped portion having an air bleeder hole for heat radiation in a plurality of places in an internal area thereof, and straight-linear middle crimped portions; and a cooling medium containing gel, sodium polyacrylate, ammonium sulfate, sodium sulfate, glycerin, water, fungicide, and the like, is accommodated in said exterior element such that migration thereof is restricted by said outer edge crimped portion, partially crimped portions in the internal area thereof, and middle crimped portions, whereby migration of the cooling medium is restricted by said partially crimped portion and middle crimped portion, resulting in uneven distribution being eliminated; by an air bleeder hole provided in said respective partially crimped portions, heat radiation is satisfactorily performed, which allows a sufficient cooling effect for the body to be exhibited at the time of sleeping, or the like; and reversible use by front/back reversing of said exterior element is also allowed.

The invention as stated in Claim 19 can provide a human body cooling apparatus which, with substantially the same configuration as that of the invention as stated in Claim 18, and having a size selected from various sizes such as a bed sheet size, a pillow size, a half size, i.e., a middle size between these sizes, further, a car seat size, a floor cushion size, a wine bottle heat insulating seat size, and the like, can exhibit a sufficient cooling effect for the entire body, the head, or a part of the body at the time of sleeping, or the like, in the same manner as with the invention as stated in Claim 18, and is also capable of reversible use by front/back reversing of an exterior element.

The invention as stated in Claim 20 can provide a human body cooling apparatus which, with substantially the same configuration as that of the invention as stated in Claim 19, and a configuration having a partially crimped portion with a toric shape, square shape, triangular shape, oval shape, or the like, in a plurality of places that has an air bleeder hole for heat radiation with a shape selected from a circular shape, square shape, triangular shape, oval shape, and the like, can exhibit a sufficient cooling effect for the entire body, the head, or a part of the body at the time of sleeping, or the like, in the same manner as with the invention as stated in Claim 19, and is also capable of reversible use by front/back reversing of said exterior element.

### Brief Description of the Drawings

FIG. 1 is a top view of a human body cooling apparatus pertaining to an Embodiment 1 of the first invention;
FIG. 2 is a side view of the human body cooling apparatus pertaining to the present Embodiment 1;
FIG. 3 is an enlarged sectional view of the human body cooling apparatus pertaining to the present Embodiment 1;
FIG. 4 is a drawing showing an example of a crimp pattern of backing cloths in the human body cooling apparatus pertaining to the present Embodiment 1;
FIG. 5 is a schematic perspective view illustrating the mode of use of the human body cooling apparatus pertaining to the present Embodiment 1;
FIG. 6 is an explanatory drawing for the cooling function of the human body cooling apparatus pertaining to the present Embodiment 1;
FIG 7 is a drawing showing a second example of a crimp pattern of backing cloths in the human body cooling apparatus pertaining to the present Embodiment 1;
FIG. 8 is a drawing showing a third example of a crimp pattern of backing cloths in the human body cooling apparatus pertaining to the present Embodiment 1;
FIG. 9 is a drawing showing a fourth example of a crimp pattern of backing cloths in the human body cooling apparatus pertaining to the present Embodiment 1;
FIG. 10 is a drawing showing a fifth example of a crimp pattern of backing cloths in the human body cooling apparatus pertaining to the present Embodiment 1;
FIG. 11 is a drawing showing a sixth example of a crimp pattern of backing cloths in the human body cooling apparatus pertaining to the present Embodiment 1;
FIG. 12 is a top view of a human body cooling apparatus pertaining to an Embodiment 2 of the first invention;
FIG. 13 is a schematic perspective view illustrating the mode of use of the human body cooling apparatus pertaining to the present Embodiment 2;
FIG. 14 is a top view of a human body cooling apparatus pertaining to an Embodiment 3 of the first invention; and
FIG. 15 is a schematic perspective view illustrating the mode of use of the human body cooling apparatus pertaining to the present Embodiment 3.
FIG. 16 is a top view of a human body cooling apparatus pertaining to an Embodiment 1 of the first invention;
FIG. 17 is a side view of the human body cooling apparatus pertaining to the present Embodiment 1;
FIG. 18 is an enlarged sectional view of the human body cooling apparatus pertaining to the present Embodiment 1;
FIG. 19 is a drawing showing an example of a crimp pattern of backing cloths in the human body cooling apparatus pertaining to the present Embodiment 1;
FIG. 20 is a schematic perspective view illustrating the mode of use of the human body cooling apparatus pertaining to the present Embodiment 1;
FIG. 21 is an explanatory drawing for the cooling function of the human body cooling apparatus pertaining to the present Embodiment 1;
FIG. 22 is a drawing showing a second example of a crimp pattern of backing cloths in the human body cooling apparatus pertaining to the present Embodiment 1;
FIG. 23 is a drawing showing a third example of a crimp pattern of backing cloths in the human body cooling apparatus pertaining to the present Embodiment 1;
FIG. 24 is a drawing showing a fourth example of a crimp pattern of backing cloths in the human body cooling apparatus pertaining to the present Embodiment 1;
FIG. 25 is a drawing showing a fifth example of a crimp pattern of backing cloths in the human body cooling apparatus pertaining to the present Embodiment 1;
FIG. 26 is a drawing showing a sixth example of a crimp pattern of backing cloths in the human body cooling apparatus pertaining to the present Embodiment 1;
FIG. 27 is a top view of a human body cooling apparatus pertaining to an Embodiment 2 of the first invention;
FIG. 28 is a schematic perspective view illustrating the mode of use of the human body cooling apparatus pertaining to the present Embodiment 2;
FIG. 29 is a top view of a human body cooling apparatus pertaining to an Embodiment 3 of the first invention; and
FIG. 30 is a schematic perspective view illustrating the mode of use of the human body cooling apparatus pertaining to the present Embodiment 3.
FIG. 31 is a top view of the human body cooling apparatus pertaining to an Embodiment 1 of a third invention;
FIG. 32 is a side view of the human body cooling apparatus pertaining to the present Embodiment 1;
FIG. 33 is a partially enlarged sectional view of the human body cooling apparatus pertaining to the present Embodiment 1;
FIG. 34 is a schematic top view illustrating the mode of use of the human body cooling apparatus pertaining to the present Embodiment 1;
FIG. 35 is an explanatory drawing of the cooling function of the human body cooling apparatus pertaining to the present Embodiment 1;
FIG. 36 is a partially enlarged view illustrating a specific example of a partially crimped portion and air bleeder hole in the human body cooling apparatus pertaining to the present Embodiment 1;
FIG. 37 is a partially enlarged view illustrating another specific example of a partially crimped portion and air bleeder hole in the human body cooling apparatus pertaining to the present Embodiment 1;
FIG. 38 is a partially enlarged view illustrating still another specific example of a partially crimped portion and air bleeder hole in the human body cooling apparatus pertaining to the present Embodiment 1;
FIG. 39 is a partially enlarged view illustrating still another specific example of a partially crimped portion and air bleeder hole in the human body cooling apparatus pertaining to the present Embodiment 1;
FIG. 40 is a top view of a human body cooling apparatus pertaining to an Embodiment 2 of the third invention;
FIG. 41 is a schematic top view illustrating the human body cooling apparatus pertaining to the present Embodiment 2 and the mode of use thereof;
FIG. 42 is a top view of the human body cooling apparatus pertaining to an Embodiment 3 of the third invention; and
FIG. 43 is a schematic top view illustrating the mode of use of the human body cooling apparatus pertaining to the present Embodiment 3.

### Description of Symbols

1: Human body cooling apparatus
1A: Human body cooling apparatus
1B: Human body cooling apparatus
2: Covering member
3 : Front cloth
4: Backing cloth
5: Cooling medium
6: Four-side outer edge crimped portion
7: Partially crimped portion
8: Partially crimped portion
9: Partially crimped portion
10: Partially crimped portion
11: Partially crimped portion
12: Partially crimped portion
13: Partially crimped portion
14: Exterior element
15: Bed
16: Sheet
201: Human body cooling apparatus
201A: Human body cooling apparatus
201B: Human body cooling apparatus
203: Front cloth
204: Backing cloth
205: Cooling medium
206: Four-side outer edge crimped portion
207: Partially crimped portion
208: Partially crimped portion
209: Partially crimped portion
210: Partially crimped portion
211: Partially crimped portion
212: Partially crimped portion
213: Partially crimped portion
214: Exterior element
214a: Side cloth
215: Bed
216: Sheet
301: Human body cooling apparatus
301A: Human body cooling apparatus
301B: Human body cooling apparatus
302: Exterior element
302a: Side cloth
303: Front cloth
304: Backing cloth
305: Cooling medium
306: Outer edge crimped portion
307: Partially crimped portion
308: Air bleeder hole
309: Middle crimped portion
310: Bed
311: Sheet
312: Pillow
M: Sleeping person

### Best Mode for Carrying Out the Invention

It is an object of the first invention to provide a human body cooling apparatus which, in any sizes ranging from a long size, such as a bed sheet size, to a relatively small size, such as a pillow size, can suppress uneven distribution due to the migratability of the cooling medium and exhibit a sufficient cooling effect for the body.

The human body cooling apparatus of the first invention has achieved the aforementioned object with a configuration including an exterior element in which two covering members in the shape of a rectangular seat each having a front cloth made of fabric, and a backing cloth which is weldable by high-frequency heating, of a size selected from various sizes such as, for example, a bed sheet size, a pillow size, a half size, i.e., a middle size between these sizes, further, a car seat size, a floor cushion size, a wine bottle heat insulating seat size, and the like, are put one upon another to provide a flat form, said backing cloths being superposed on each other; and a cooling medium containing gel accommodated in said exterior element such that migration thereof is restricted by a four-side outer edge crimped portion and a partially crimped portion in an internal area thereof that are produced by high-frequency welding of the backing cloths in said two covering members.

It is an object of the second invention to provide a human body cooling apparatus which, in any sizes ranging from a long size, such as a bed sheet size, to a relatively small size, such as a pillow size, can suppress uneven distribution due to the migratability of the cooling medium and exhibit a sufficient cooling effect for the body, and also allows reversible use.

The human body cooling apparatus of the second invention has achieved the aforementioned object with a configuration including an exterior element in which two covering members in the shape of a rectangular seat each having a font cloth formed of a material selected from cotton fiber, polyester, rayon, hemp, raised fabric, crepe, striped crepe, towel fabric, and the like, and a backing cloth formed of a non-water absorbent material, such as polyvinyl chloride, or the like, which is weldable by high-frequency heating, of a size selected from various sizes such as, for example, a bed sheet size, a pillow size, a half size, i.e., a middle size between these sizes, further, a car seat size, a floor cushion size, a wine bottle heat insulating seat size, and the like, are put one upon another to provide a flat form, said backing cloths being superposed on each other; and a cooling medium containing gel, sodium polyacrylate, ammonium sulfate, sodium sulfate, glycerin, water, fungicide, and the like, accommodated in said exterior element such that migration thereof is restricted by a four-side outer edge crimped portion and a partially crimped portion in an internal area thereof that are produced by high-frequency welding of the backing cloths in said two covering members; said exterior element being capable of reversible use by front/back reversing it.

It is an object of the third invention to provide a human body cooling apparatus which, in any sizes ranging from a long size, such as a bed sheet size, to a relatively small size, such as a pillow size, can suppress uneven distribution due to the migratability of the cooling medium and exhibit a sufficient cooling effect for the body, being also excellent in function of radiating the heat, and also allows reversible use.

The human body cooling apparatus of the third invention has achieved the aforementioned object with a configuration including an exterior element in which two covering members in the shape of a rectangular seat each having a front cloth formed of a material selected from cotton fiber, polyester, rayon, hemp, raised fabric, crepe, striped crepe, towel fabric, and the like, and a backing cloth formed of a non-water absorbent material, such as polyvinyl chloride, or the like, which is weldable by high-frequency heating are put one upon another to provide a flat form, said backing cloths being superposed on each other and the backing cloths being high-frequency welded for formation of an outer edge crimped portion, a toric partially crimped portion having an air bleeder hole for heat radiation in a plurality of places in an internal area thereof, and straight-linear middle crimped portions; and a cooling medium containing gel, sodium polyacrylate, ammonium sulfate, sodium sulfate, glycerin, water, fungicide, and the like, accommodated in said exterior element such that migration thereof is restricted by said outer edge crimped portion, partially crimped portions in the internal area thereof, and middle crimped portions; said exterior element being capable of reversible use by front/back reversing it.

### Embodiments of First Invention:

Hereinbelow, human body cooling apparatuses pertaining to embodiments of the first invention will be described in details with reference to the drawings.

### Embodiment 1:

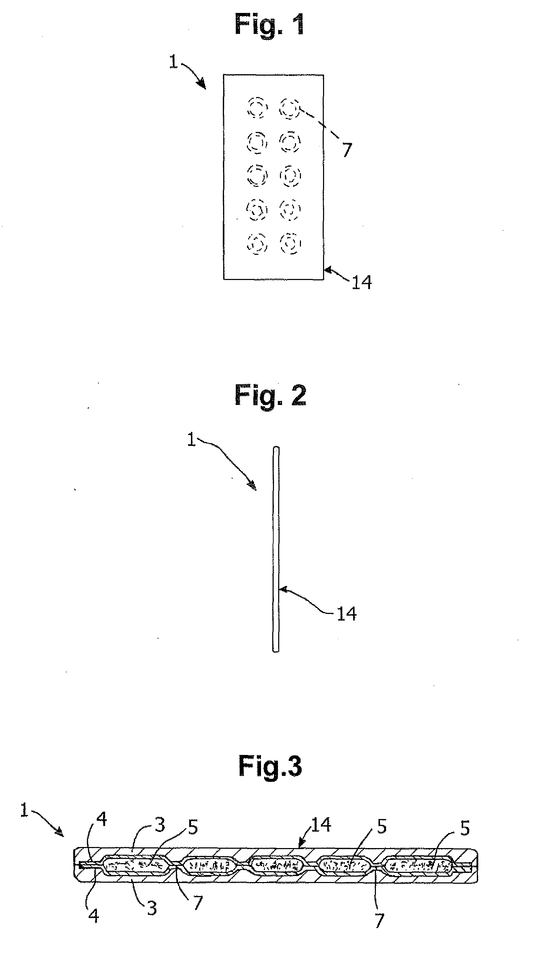

The human body cooling apparatus 1 pertaining to the present Embodiment 1 will be described with reference to FIG. 1 to FIG. 11.

As shown in FIG. 1 to FIG. 3, the human body cooling apparatus 1 pertaining to the present Embodiment 1 includes an exterior element 14 including two covering members in the shape of a rectangular seat that are each constituted by a front cloth 3 made of a cloth formed of, for example, cotton fiber, and a backing cloth 4 which is weldable by high-frequency heating, being made of, for example, polyethylene, and which are each produced by superposing the front cloth 3 and the backing cloth 4 on each other, of a size selected from various sizes such as, for example, a bed sheet size (of, for example, a width of 90 cm and a length of 180 cm), a pillow size, a half size, i.e., a middle size between these sizes, further, a car seat size, a floor cushion size, a wine bottle heat insulating seat size, and the like, the two covering members being put one upon another to provide a flat form, said backing cloths 4 being superposed on each other, the two covering members being combined with each other in a front and back relationship, and said backing cloths 4 being superposed on each other in the mutually opposed state in order to accommodate a cooling medium 5 containing gel inside the backing cloths 4 by high-frequency welding them to each other such that it is hermetically sealed in a predetermined crimp pattern for restriction of migration of the cooling medium 5. The outer surfaces of said both front cloths 3 are subjected to anti-fungal treatment.

Examples of a composition of said cooling medium 5 containing gel include a composition consisting of a gel, a water-absorbent polymer, paraben, a fungicide, glycerin, and water.

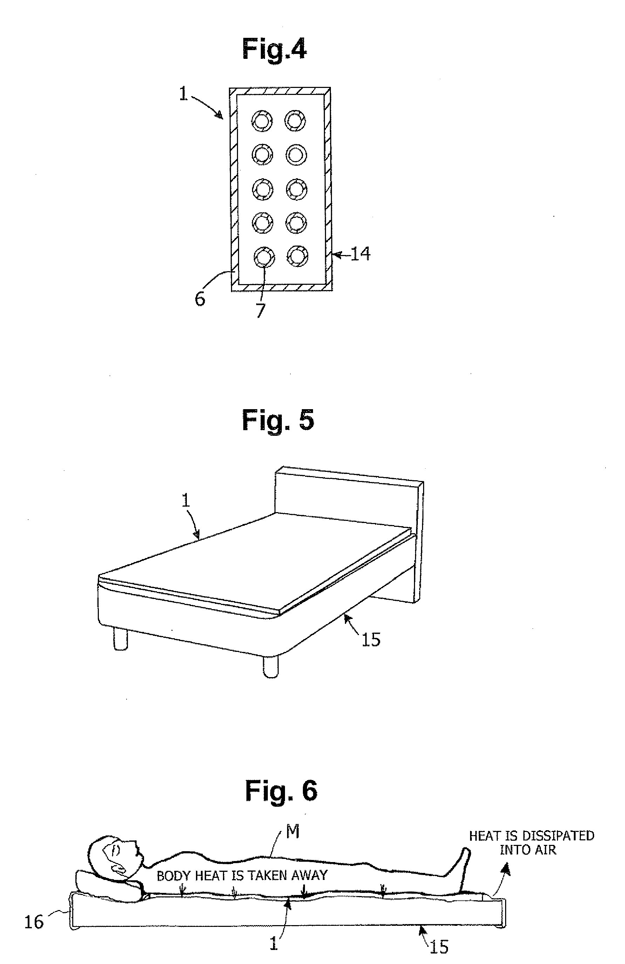

FIG. 4 shows an example of a crimp pattern for said backing cloths 4, and this example pattern is constituted by a four-side outer edge crimped portion 6 of said backing cloths 4, and a toric partially crimped portion 7 in the internal area thereof, said toric partially crimped portion 7 being interspersed in, for example, ten places in an arrangement along the lengthwise and lateral directions.

Next, with reference to FIG. 5, the mode of use, and the like, of the human body cooling apparatus 1 pertaining to the present Embodiment 1 will be described.

As shown in FIG. 5, for example, the human body cooling apparatus 1 pertaining to the present Embodiment 1 is used in such a mode as that in which it is placed on the top of a sheet 16 on a bed (or a futon mattress) 15 for use in sleeping, or the like, and a sleeping person M lies on said human body cooling apparatus 1 for sleep.

At this time, because the toric partially crimped portion 7 is provided in the internal area of the four-side outer edge crimped portion 6, the migratability of the cooling medium 5 containing gel in the inside of this human body cooling apparatus 1 will be moderately suppressed, even if the weight of the sleeping person M acts on the human body cooling apparatus 1.

In addition, as shown in FIG. 6, the human body cooling apparatus 1 is comfortably fitted to the body, and due to the temperature difference between the room temperature and the body temperature of the sleeping person M, the cooling medium 5 takes away the body heat of the back, waist, foot, and the like, of the sleeping person M, which enhances the cooling effect. In addition, from the cooling medium 5 in the inside of the human body cooling apparatus 1 on which the body of the sleeping person M does not directly rest, the heat is dissipated into the atmosphere in the room.

Thus, the human body cooling apparatus 1 pertaining to the present Embodiment 1 has excellent effects of giving a good feeling upon use to the sleeping person M, and moderately cooling the body temperature of the sleeping person M from the start of sleeping to awaking in the high temperature season, thus realizing comfortable sleep.

For schematic comparison between the back skin temperature at the time of sleeping of the sleeping person M for 60 min. when the human body cooling apparatus 1 pertaining to the present Embodiment 1 is used, and that when the same is not used, temperature measurement has been carried out at an ambient temperature of 30 deg. C. As a result of this, it has been revealed that, when the human body cooling apparatus 1 is used, the skin temperature at the back of the sleeping person M is suppressed to a rather low one except for the area of the nape where the skin temperature is rather high, while, when the human body cooling apparatus 1 is not used, the skin temperature at the back of the sleeping person M is raised in substantially the entire area of the back.

In addition, for schematic comparison between the back skin temperature at the time of sleeping of the sleeping person M for 240 min. when the human body cooling apparatus 1 pertaining to the present Embodiment 1 is used, and that when the same is not used, temperature measurement has been carried out at an ambient temperature of 30 deg. C.

As a result of this, it has been revealed that, when the human body cooling apparatus 1 is used, the skin temperature at the back of the sleeping person M from the start of sleeping until 240 min. has elapsed is held all the time to a skin temperature 2 to 3 deg. lower than that when the human body cooling apparatus 1 is not used.

As can be seen from the results of the temperature measurement as stated above, the human body cooling apparatus 1 pertaining to the present Embodiment 1 exhibits an excellent cooling effect during the sleep of the sleeping person M.

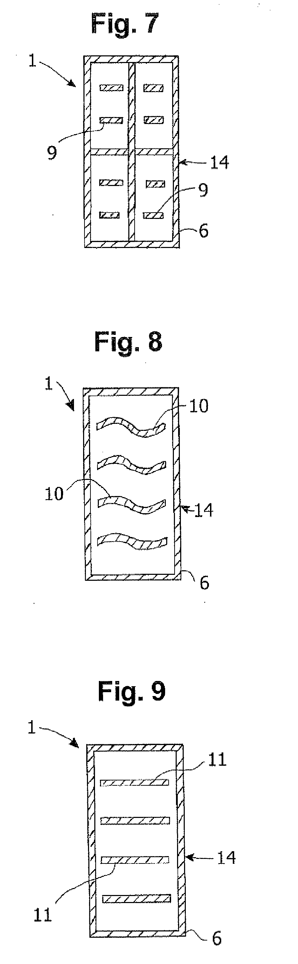

Next, with reference to FIG. 7 to FIG 11, second to sixth examples of a crimp pattern of the backing cloths 4 in the human body cooling apparatus 1 pertaining to the present Embodiment 1 will be described.

FIG. 7 illustrates the second example of a crimp pattern of the backing cloths 4 in the human body cooling apparatus 1 pertaining to the present Embodiment 1, in which, in addition to the aforementioned four-side outer edge crimped portion 6, a cross-shaped partially crimped portion 8 connecting between the middle portions of the respective opposed sides in the four-side outer edge crimped portion 6, and plural rectangular partially crimped portions 9 interspersed in the respective four areas defined by this cross-shaped partially crimped portion 8 are formed.

FIG. 8 illustrates the third example of a crimp pattern of the backing cloths 4 in the human body cooling apparatus 1 pertaining to the present Embodiment 1, in which, in addition to the aforementioned four-side outer edge crimped portion 6, a plurality of corrugated partially crimped portions 10 interspersed in the internal area thereof are formed.

FIG. 9 illustrates the fourth example of a crimp pattern of the backing cloths 4 in the human body cooling apparatus 1 pertaining to the present Embodiment 1, in which, in addition to the aforementioned four-side outer edge crimped portion 6, a plurality of rectangular partially crimped portions 11 interspersed in the internal area thereof are formed.

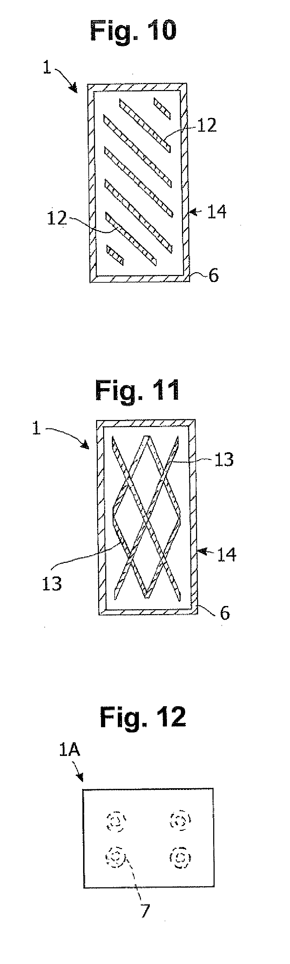

FIG. 10 illustrates the fifth example of a crimp pattern of the backing cloths 4 in the human body cooling apparatus 1 pertaining to the present Embodiment 1, in which, in addition to the aforementioned four-side outer edge crimped portion 6, a plurality of partially crimped portions 12 being arranged in parallel to one another in an inclined disposition in the internal area thereof to exhibit a substantially rectangular space occupying configuration as a whole are formed.

FIG. 11 illustrates the sixth example of a crimp pattern of the backing cloths 4 in the human body cooling apparatus 1 pertaining to the present Embodiment 1, in which, in addition to the aforementioned four-side outer edge crimped portion 6, a plurality of partially crimped portions 13 being interspersed and arranged in a pattern of crosses in the internal area thereof, each cross exhibiting a substantially rectangular space occupying configuration as a whole, are formed.

Needless to say, in the crimp patterns as shown in FIG. 7 to FIG. 11, the respective numbers of partially crimped portions 9 to 13 formed are not limited to that as given in the respective examples illustrated.

Also with the human body cooling apparatus 1 which adopts the respective crimp patterns as shown in FIG. 7 to FIG. 11, the same functional effect as that with the aforedescribed human body cooling apparatus 1 which adopts the crimp pattern as shown in FIG. 4 can be obtained, respectively.

### Embodiment 2:

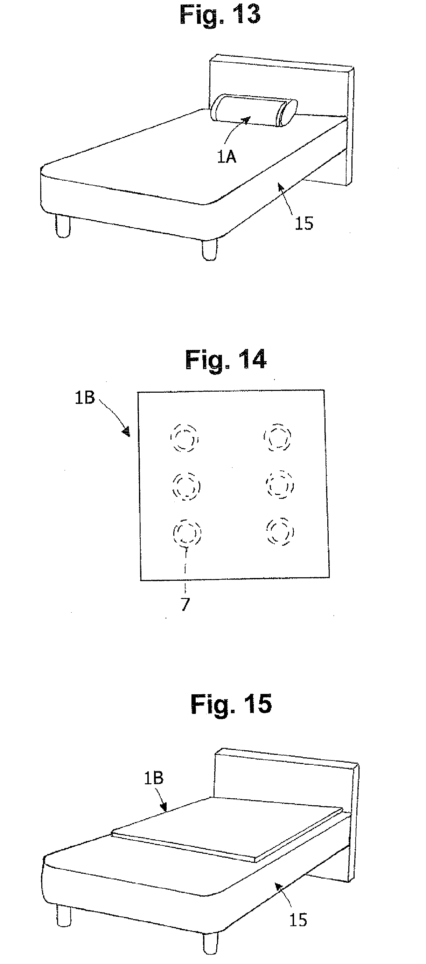

The human body cooling apparatus 1A pertaining to the present Embodiment 2 will be described with reference to FIG. 12 and FIG. 13.

The human body cooling apparatus 1A pertaining to the present Embodiment 2 has substantially the same configuration as that of the aforedescribed human body cooling apparatus 1, however, as shown in FIG. 12, it is configured for use with a pillow, the entire size being reduced to that of, for example, a width of 30 cm, and a length of 40 cm.

Also for this human body cooling apparatus 1A, the respective crimp patterns of the backing cloths 4 as shown in FIG. 4, and FIG. 7 to FIG. 11 can be adopted.

FIG. 13 illustrates the mode of use of the human body cooling apparatus 1A pertaining to the present Embodiment 2. According to this human body cooling apparatus 1A, the human body cooling apparatus 1A is comfortably fitted to the head of the sleeping person M, and due to the temperature difference between the room temperature and the body temperature of the sleeping person M, the cooling medium 5 takes away the body heat of the head of the sleeping person M, which enhances the cooling effect, providing the state in which the head is kept cool, and the feet is kept warm.

### Embodiment 3:

A human body cooling apparatus 1B pertaining to the present Embodiment 3 will be described with reference to FIG 14 and FIG. 15.

The human body cooling apparatus 1B pertaining to the present Embodiment 3 has substantially the same configuration as that of the aforedescribed human body cooling apparatus 1, however, as shown in FIG. 14, it is configured to provide a half size, i.e., a middle size between the sizes of said human body cooling apparatus 1 and said human body cooling apparatus 1A, the entire size being reduced to that of, for example, a width of 90 cm, and a length of 90 cm.

Also for this human body cooling apparatus 1B, the respective crimp patterns of the backing cloths 4 as shown in FIG. 4, and FIG 7 to FIG. 11 can be adopted.

FIG. 15 illustrates the mode of use of the human body cooling apparatus 1B pertaining to the present Embodiment 3, the human body cooling apparatus 1B taking away the body heat of the back, and the like, of the sleeping person M, which enhances the cooling effect, and substantially the same functional effect as that with said human body cooling apparatus 1 can be obtained.

### Embodiments of Second invention:

Hereinbelow, human body cooling apparatuses pertaining to embodiments of the second invention will be described in details with reference to the drawings. Embodiment 1:

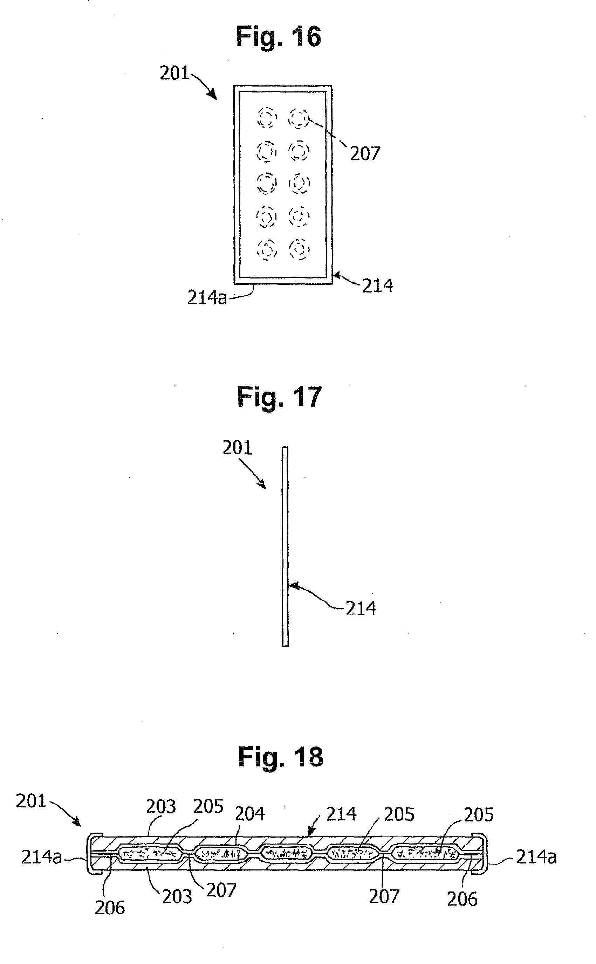

The human body cooling apparatus 201 pertaining to the present Embodiment 1 will be described with reference to FIG. 16 to FIG. 26.

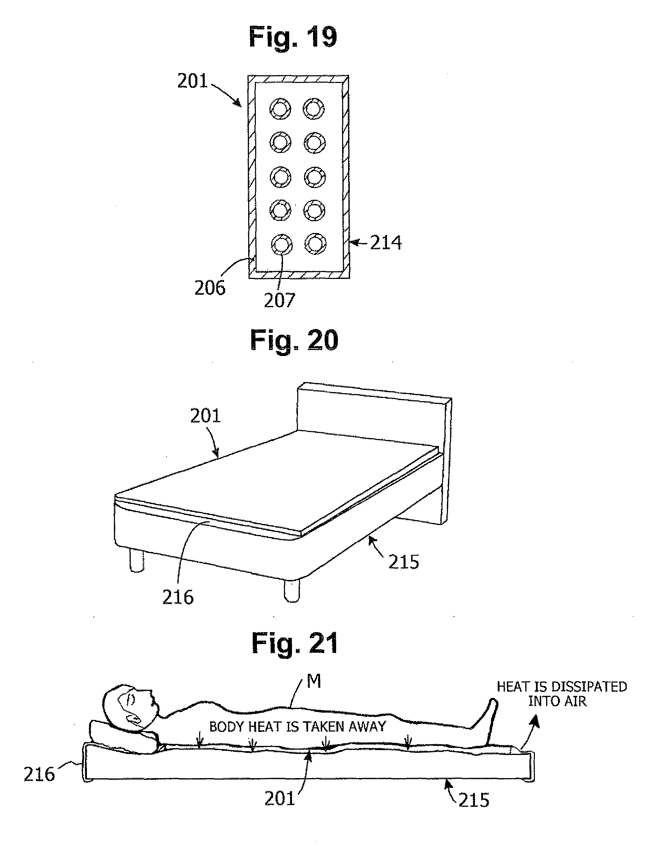

As shown in FIG. 16 to FIG. 18, the human body cooling apparatus 201 pertaining to the present Embodiment 1 includes an exterior element 214 in which one covering member in which a front cloth 203 formed of, for example, cotton fiber, polyester, rayon, hemp, raised fabric, crepe, striped crepe, towel fabric, or the like, and a backing cloth 204 formed of a non-water absorbent material, such as polyvinyl chloride (PVC), or the like, which is weldable by high-frequency heating are superposed on each other, and the other covering member which is constituted by the front cloth 203 and the backing cloth 204, being configured in the same manner, are put upon one another with said backing cloths 204 being disposed opposed to each other and being high-frequency welded, and a cooling medium 205 containing gel which is accommodated in this exterior element 214, being hermetically sealed. FIG. 19 schematically shows a crimp pattern in the human body cooling apparatus 201, and in FIG. 19, a side cloth 214a outside a four-side outer edge crimped portion 206, and the like are omitted.

Said exterior element 214 is provided with a shape of a rectangular seat, and a size selected from various sizes such as, for example, a bed sheet size (of, for example, a width of 90 cm and a length of 180 cm), a pillow size, a half size, i.e., a middle size between these sizes, further, a car seat size, a floor cushion size, a wine bottle heat insulating seat size, and the like.

Said cooling medium 205 is hermetically sealed and accommodated in the exterior element 214 with said both backing cloths 204 being high-frequency welded to each other in a predetermined crimp pattern as described later, such as that constituted by the four-side outer edge crimped portion 206, a partially crimped portion 207, and the like, for restriction of migration of the cooling medium 205. The outer surfaces of said both front cloths 203 are subjected to anti-fungal treatment. In addition, in the outer edge portion outside said four-side outer edge crimped portion 206, the side cloth 214a is disposed (by hemming), being stitched by sewing over the entire periphery.

Examples of a composition of said cooling medium 205 containing gel include a composition consisting of a gel, a sodium polyacrylate, ammonium sulfate, sodium sulfate, glycerin, water, a fungicide, and the like.

FIG. 19 shows an example of a crimp pattern for said backing cloths 204, and this example pattern is constituted by the four-side outer edge crimped portion 206 of said backing cloths 204, and the toric partially crimped portion 207 in the internal area thereof, said toric partially crimped portion 207 being interspersed in, for example, ten places in an arrangement along the lengthwise and lateral directions.

Next, with reference to FIG. 20, the mode of use, and the like, of the human body cooling apparatus 201 pertaining to the present Embodiment 1 will be described.

As shown in FIG. 20, for example, the human body cooling apparatus 201 pertaining to the present Embodiment 1 is used in such a mode as that in which it is placed on the top of a sheet 216 on a bed (or a futon mattress) 15 for use in sleeping, or the like, and a sleeping person M lies on said human body cooling apparatus 201 for sleep.

At this time, because the toric partially crimped portion 207 is provided in the internal area of the four-side outer edge crimped portion 206, the migratability of the cooling medium 205 containing gel in the inside of this human body cooling apparatus 201 will be moderately suppressed, even if the weight of the sleeping person M acts on the human body cooling apparatus 201.

In addition, as shown in FIG. 21, the human body cooling apparatus 201 is comfortably fitted to the body, and due to the temperature difference between the room temperature and the body temperature of the sleeping person M, the cooling medium 205 takes away the body heat of the back, waist, foot, and the like, of the sleeping person M, which enhances the cooling effect. In addition, from the cooling medium 5 in the area of the human body cooling apparatus 201 on which the body of the sleeping person M does not directly rest, the heat is dissipated into the atmosphere in the room.

Thus, the human body cooling apparatus 201 pertaining to the present Embodiment has excellent effects of giving a good feeling upon use to the sleeping person M, and moderately cooling the body temperature of the sleeping person M from the start of sleeping to awaking in the high temperature season, thus realizing comfortable sleep.

For schematic comparison between the back skin temperature at the time of sleeping of the sleeping person M for 60 min. when the human body cooling apparatus 201 pertaining to the present Embodiment 1 is used, and that when the same is not used, temperature measurement has been carried out at an ambient temperature of 30 deg. C.

As a result of this, it has been revealed that, when the human body cooling apparatus 201 is used, the skin temperature at the back of the sleeping person M is suppressed to a rather low one except for the area of the nape where the skin temperature is rather high, while, when the human body cooling apparatus 201 is not used, the skin temperature at the back of the sleeping person M is raised in substantially the entire area of the back.

In addition, for schematic comparison between the back skin temperature at the time of sleeping of the sleeping person M for 240 min. when the human body cooling apparatus 201 pertaining to the present Embodiment 1 is used, and that when the same is not used, temperature measurement has been carried out at an ambient temperature of 30 deg. C.

As a result of this, it has been revealed that, when the human body cooling apparatus 201 is used, the skin temperature at the back of the sleeping person M from the start of sleeping until 240 min. has elapsed is held all the time to a skin temperature 2 to 3 deg. lower than that when the human body cooling apparatus 201 is not used.

As can be seen from the results of the temperature measurement as stated above, the human body cooling apparatus 201 pertaining to the present Embodiment 1 exhibits an excellent cooling effect during the sleep of the sleeping person M.

In addition, the human body cooling apparatus 201 pertaining to the present Embodiment 1 is also capable of reversible use by front/back reversing said exterior element 214, whereby the human body cooling apparatus 201 can withstand the use over a longer period of time.

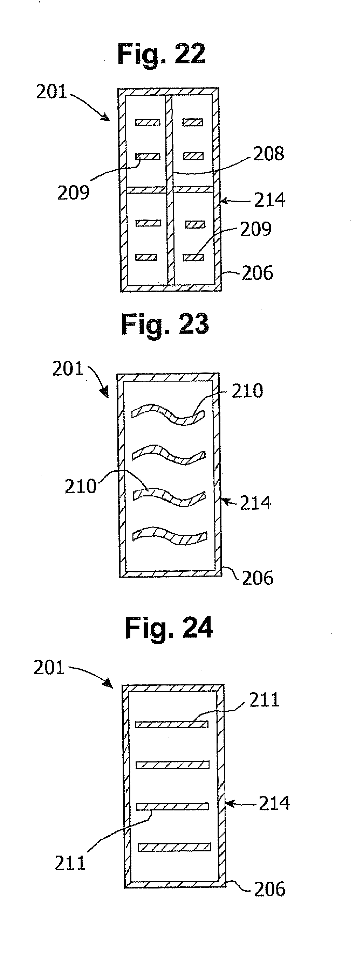

Next, with reference to FIG. 22 to FIG 26, second to sixth examples of a crimp pattern of the backing cloths 204 in the human body cooling apparatus 201 pertaining to the present Embodiment 1 will be described. FIG. 22 to FIG. 26 schematically show a crimp pattern in the respective human body cooling apparatuses 201, and in FIG. 22 to FIG. 26, the side cloth 214a outside the four-side outer edge crimped portion 206, and the like are omitted.

FIG. 22 illustrates the second example of a crimp pattern of the backing cloths 204 in the human body cooling apparatus 201 pertaining to the present Embodiment 1, in which, in addition to the aforementioned four-side outer edge crimped portion 206, a cross-shaped partially crimped portion 208 connecting between the middle portions of the opposed sides, and a plurality of rectangular partially crimped portions 209 interspersed in the respective four areas defined by this cross-shaped partially crimped portion 208 are formed.

FIG 23 illustrates the third example of a crimp pattern of the backing cloths 204 in the human body cooling apparatus 201 pertaining to the present Embodiment 1, in which, in addition to the aforementioned four-side outer edge crimped portion 206, a plurality of corrugated partially crimped portions 210 interspersed in the internal area thereof are formed.

FIG. 24 illustrates the fourth example of a crimp pattern of the backing cloths 204 in the human body cooling apparatus 201 pertaining to the present Embodiment 1, in which, in addition to the aforementioned four-side outer edge crimped portion 206, a plurality of rectangular partially crimped portions 211 interspersed in the internal area thereof are formed.

FTG. 25 illustrates the fifth example of a crimp pattern of the backing cloths 204 in the human body cooling apparatus 201 pertaining to the present Embodiment 1, in which, in addition to the aforementioned four-side outer edge crimped portion 206, a plurality of partially crimped portions 212 being arranged in parallel to one another in an inclined disposition in the internal area thereof to exhibit a substantially rectangular space occupying configuration as a whole are formed.

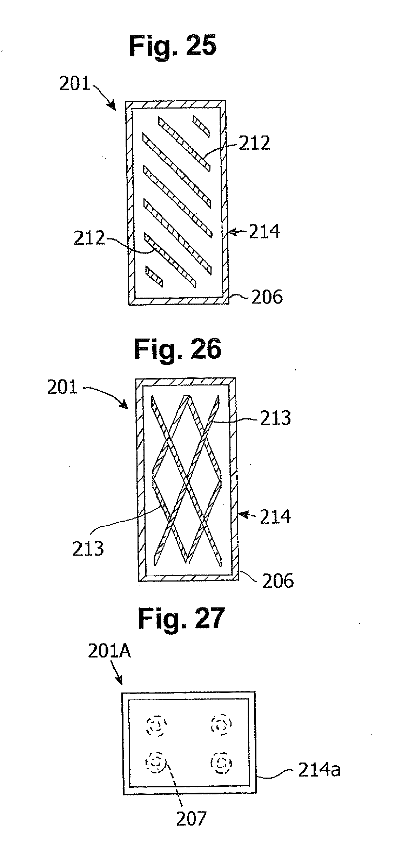

FIG 26 illustrates the sixth example of a crimp pattern of the backing cloths 204 in the human body cooling apparatus 201 pertaining to the present Embodiment 1, in which, in addition to the aforementioned four-side outer edge crimped portion 206, a plurality of partially crimped portions 213 being interspersed and arranged in a pattern of crosses in the internal area thereof, each cross exhibiting a substantially rectangular space occupying configuration as a whole, are formed.

Needless to say, in the crimp patterns as shown in FIG. 22 to FIG. 26, the respective numbers of partially crimped portions 209 to 213 formed are not limited to that as given in the respective examples illustrated.

Also with the human body cooling apparatus 1 which adopts the respective crimp patterns as shown in FIG. 22 to FIG. 26, the same functional effect as that with the aforedescribed human body cooling apparatus 201 which adopts the crimp pattern as shown in FIG 19 can be obtained, respectively.

In addition, the human body cooling apparatus 201A pertaining to the present Embodiment 1 is also capable of reversible use by front/back reversing this human body cooling apparatus 201A, whereby the human body cooling apparatus 201A can withstand the use over a longer period of time.

### Embodiment 2:

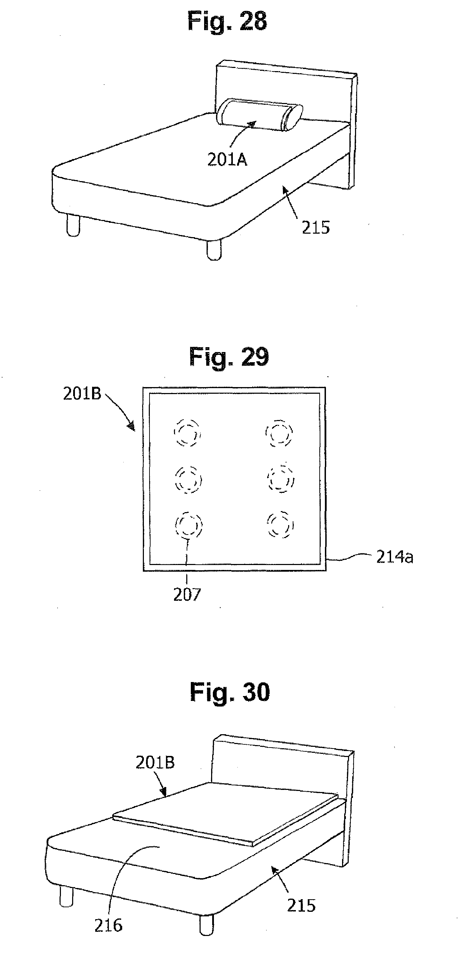

The human body cooling apparatus 201A pertaining to the present Embodiment 2 will be described with reference to FIG 27 and FIG 28.

The human body cooling apparatus 201A pertaining to the present Embodiment 2 has substantially the same configuration as that of the aforedescribed human body cooling apparatus 201, however, as shown in FIG. 27, it is configured for use with a pillow, the entire size being reduced to that of, for example, a width of 30 cm, and a length of 40 cm.

Also for this human body cooling apparatus 201A, the respective crimp patterns of the backing cloths 204 as shown in FIG. 19 and FIG. 22 to FIG. 26 can be adopted.

FIG. 28 illustrates the mode of use of the human body cooling apparatus 201A pertaining to the present Embodiment 2. According to this human body cooling apparatus 201A, the human body cooling apparatus 201A is comfortably fitted to the head of the sleeping person M, and due to the temperature difference between the room temperature and the body temperature of the sleeping person M, the cooling medium 205 takes away the body heat of the head of the sleeping person M, which enhances the cooling effect, providing the state in which the head is kept cool, and the feet is kept warm.

### Embodiment 3:

A human body cooling apparatus 201 B pertaining to the present Embodiment 3 will be described with reference to FIG. 29 and FIG 30.

The human body cooling apparatus 20 1 B pertaining to the present Embodiment 3 has substantially the same configuration as that of the aforedescribed human body cooling apparatus 201, however, as shown in FIG. 29, it is configured to provide a half size, i.e., a middle size between the sizes of said human body cooling apparatus 201 and said human body cooling apparatus 201A, the entire size being reduced to that of, for example, a width of 90 cm, and a length of 90 cm.

Also for this human body cooling apparatus 201B, the respective crimp patterns of the backing cloths 204 as shown in FIG. 19 and FIG. 22 to FIG. 26 can be adopted.

FIG. 30 illustrates the mode of use of the human body cooling apparatus 201B pertaining to the present Embodiment 3, the human body cooling apparatus 201B taking away the body heat of the back, and the like, of the sleeping person M, which enhances the cooling effect, and substantially the same functional effect as that with said human body cooling apparatus 201 can be obtained.

In addition, the human body cooling apparatus 201B pertaining to the present Embodiment 2 is also capable of reversible use by front/back reversing this human body cooling apparatus 201B, whereby the human body cooling apparatus 201B can withstand the use over a longer period of time.

The aforementioned human body cooling apparatus 201, human body cooling apparatus 201A, and human body cooling apparatus 201B can each give a chill feeling, however, they can be used after being cooled in a refrigerator, or being warmed with a microwave oven.

### Embodiments of Third invention:

Hereinbelow, human body cooling apparatuses pertaining to embodiments of the third invention will be described in details with reference to the drawings.

### Embodiment 1:

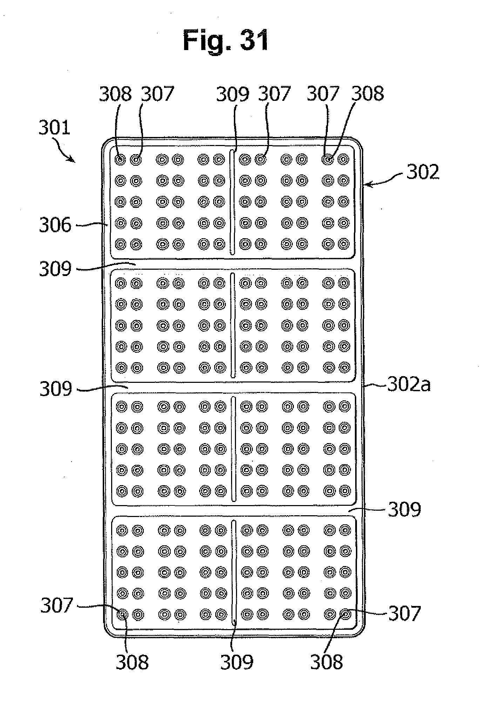

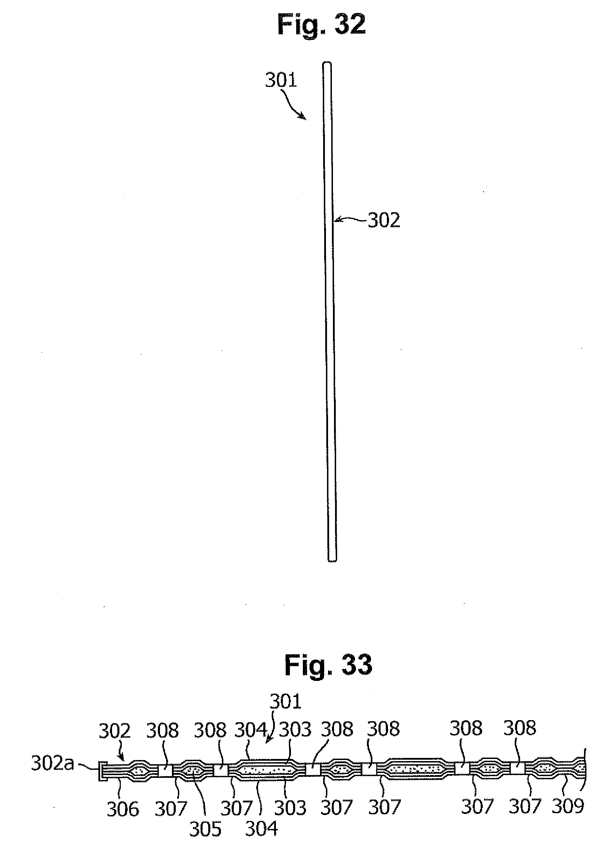

The human body cooling apparatus 301 pertaining to the present Embodiment 1 will be described with reference to FIG. 31 to FIG. 39.

As shown in FIG 31 to FIG. 33, the human body cooling apparatus 301 pertaining to the present Embodiment 1 includes an exterior element 302 in which one covering member in which a front cloth 303 formed of, for example, cotton fiber, polyester, rayon, hemp, raised fabric, crepe, striped crepe, towel fabric, or the like, and a backing cloth 304 formed of a non-water absorbent material, such as polyvinyl chloride (PVC), or the like, which is weldable by high-frequency heating are superposed on each other, and the other covering member which is constituted by the front cloth 303 and the backing cloth 304, being configured in the same manner, are put upon one another with said backing cloths 304 being disposed opposed to each other and being high-frequency welded, and a cooling medium 305 containing gel which is accommodated in this exterior element 302, being hermetically sealed.

Said exterior element 302 is provided with a shape of a rectangular seat, and a size selected from various sizes such as, for example, a bed sheet size (of, for example, a width of 90 cm and a length of 180 cm), a pillow size, a half size, i.e., a middle size between the sizes of these sizes, further, a car seat size, a floor cushion size, a wine bottle heat insulating seat size, and the like.

Said cooling medium 305 is hermetically sealed and accommodated in the exterior element 302 with said both backing cloths 304 being high-frequency welded to each other in a predetermined crimp pattern in respective crimped portions constituted by an outer edge crimped portion 306, a number of partially crimped portions 307, which are, for example, toric, having an air bleeder hole (through-hole) 308 for heat radiation, which is, for example, circular, and a straight-linear middle crimped portion 309 for restriction of migration of the cooling medium 305.

The outer surfaces of said both front cloths 303 are subjected to anti-fungal treatment. In addition, in the outer edge portion outside said four-side outer edge crimped portion 306, the side cloth 302a is disposed (by hemming), being stitched by sewing over the entire periphery.

Examples of a composition of said cooling medium 305 containing gel include a composition consisting of a gel, a sodium polyacrylate, ammonium sulfate, sodium sulfate, glycerin, water, a fungicide, and the like.

As shown in FIG. 31, said partially crimped portion 307 in the present Embodiment 1 is provided in twelve places in the lateral direction and twenty places in the longitudinal direction over the entire area of the exterior element 302, in two hundred and forty places in total. The air bleeder hole 308 is provided in each partially crimped portion 307 in the shape of a circle.

In order to form said partially crimped portion 307 and air bleeder hole 308, two hundred and forty partially crimped portions 307 in total are first formed by high-frequency welding in the exterior element 302, then, in the middle portion of each partially crimped portion 307, the air bleeder hole 308 is produced also by high-frequency welding.

As shown in FIG. 31, in the present Embodiment 1, a desired number of said middle crimped portions 309 are formed with a spacing along the lengthwise and lateral directions in the exterior element 302.

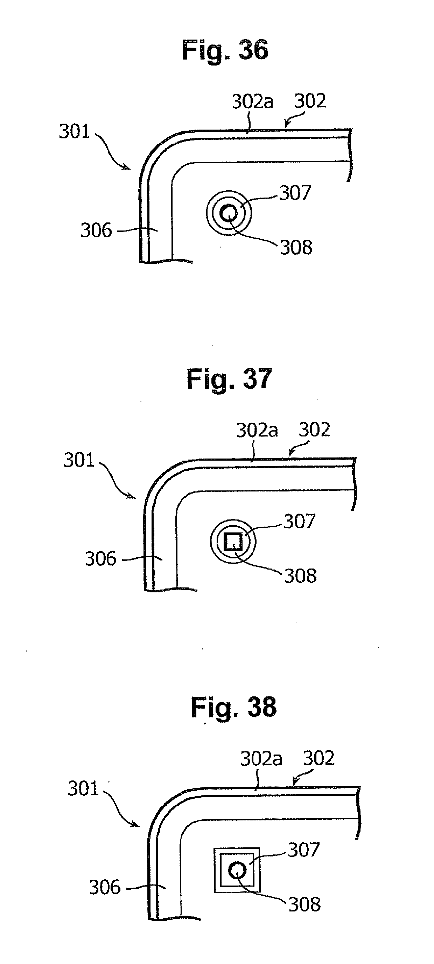

Next, specific examples of said partially crimped portion 307 and air bleeder hole 308 in the human body cooling apparatus 301 pertaining to the present Embodiment 1 will be described with reference to FIG. 36 to FIG. 39.

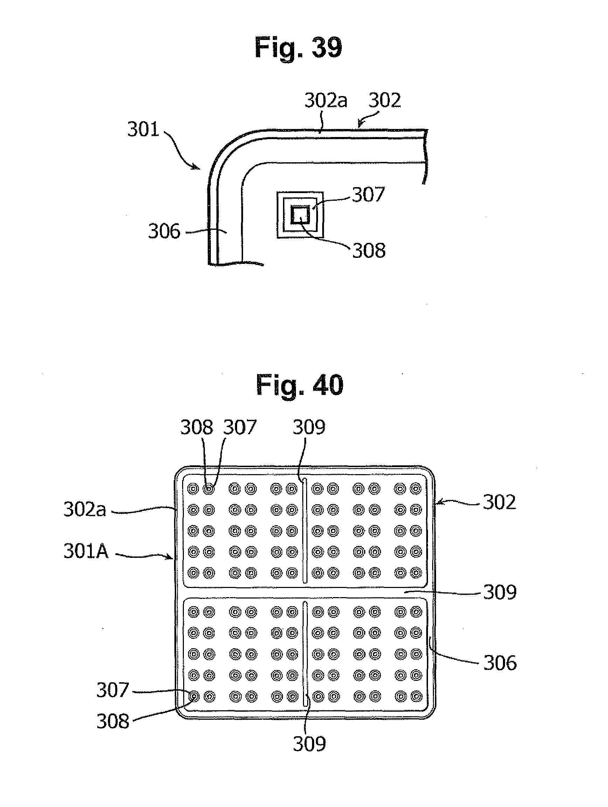

FIG. 36 gives an example in which a toric partially crimped portion 307 and a circular air bleeder hole 308 are provided in said exterior element 302; FIG. 37 gives an example in which a toric partially crimped portion 307 and a quadrangular air bleeder hole 308 are provided in said exterior element 302; FIG. 38 gives an example in which a quadrangular partially crimped portion 307 and a circular air bleeder hole 308 are provided in said exterior element 302; and further, FIG. 39 gives an example in which a quadrangular partially crimped portion 307 and a quadrangular air bleeder hole 308 are provided in said exterior element 302.

Besides these, the shape of said partially crimped portion 307 can be selected from a triangular shape, an oval shape, and the like, while the shape of said air bleeder hole 308 can be selected from a triangular shape, an oval shape, and the like.

In other words, the respective shapes of said partially crimped portion 307 and air bleeder hole 308 can be selected from various combinations of shapes, and are not limited to the respective examples as shown in FIG. 36 to FIG. 39.

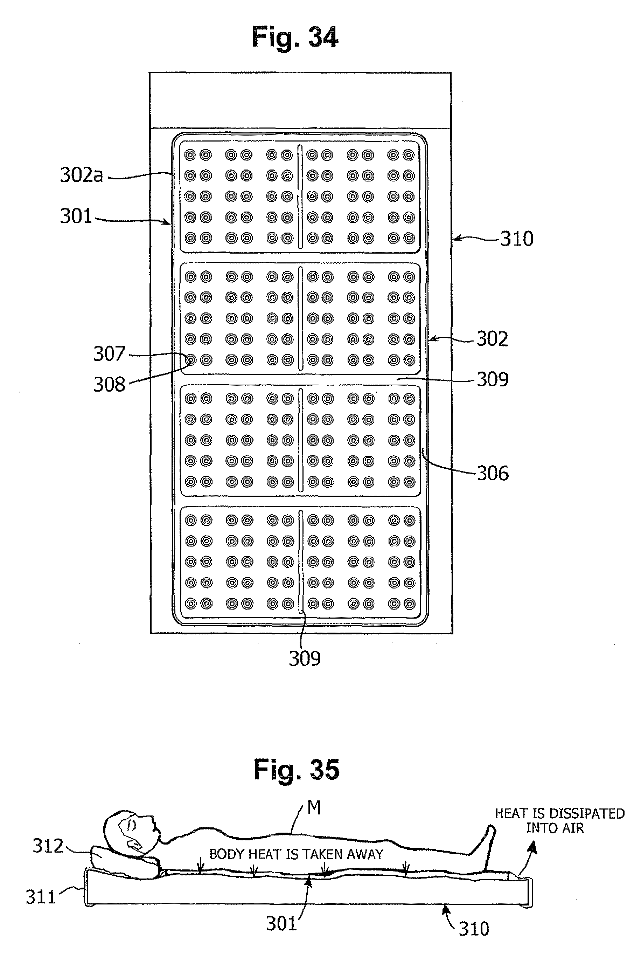

Next, with reference to FIG 34 and FIG. 35, the mode of use, and the like, of the human body cooling apparatus 301 pertaining to the present Embodiment 1 will be described.

As shown in FIG. 34 and FIG. 35, for example, the human body cooling apparatus 301 pertaining to the present Embodiment 1 is used in such a mode as that in which it is placed on the top of a sheet 311 on a bed (or a futon mattress) 310 for use in sleeping, or the like, and a sleeping person M lies on said human body cooling apparatus 301 for sleep.

At this time, because the partially crimped portion 307, which is toric, for example, is provided in great numbers in the internal area of the outer edge crimped portion 306, the migratability of the cooling medium 305 containing gel in the inside of this human body cooling apparatus 301 will be moderately suppressed, even if the weight of the sleeping person M acts on the human body cooling apparatus 301.

In addition, as shown in FIG. 35, the human body cooling apparatus 301 is comfortably fitted to the body, and due to the temperature difference between the room temperature and the body temperature of the sleeping person M, the cooling medium 305 takes away the body heat of the back, waist, foot, and the like, of the sleeping person M, which enhances the cooling effect. The heat accumulated in the cooling medium 305 is dissipated into the atmosphere in the room from the air bleeder hole 308, which is circular, for example, provided in said respective partially crimped portions 307, which are toric, for example, especially from the air bleeder hole 308, which is circular, for example, in the area of the human body cooling apparatus 301 on which the body of the sleeping person M does not directly rest. In other words, radiation of the heat from the body of the sleeping person M is satisfactorily performed.

Thus, the human body cooling apparatus 301 pertaining to the present Embodiment 1 has excellent effects of giving a good feeling upon use to the sleeping person M, and moderately cooling the body temperature of the sleeping person M from the start of sleeping to awaking in the high temperature season, thus realizing comfortable sleep.

For schematic comparison between the back skin temperature at the time of sleeping of the sleeping person M for 60 min. when the human body cooling apparatus 301 pertaining to the present Embodiment 1 is used, and that when the same is not used, temperature measurement has been carried out at an ambient temperature of 30 deg. C.

As a result of this, it has been revealed that, when the human body cooling apparatus 301 is used, the skin temperature at the back of the sleeping person M is suppressed to a rather low one except for the area of the nape where the skin temperature is rather high, while, when the human body cooling apparatus 201 is not used, the skin temperature at the back of the sleeping person M is raised in substantially the entire area of the back.

In addition, for schematic comparison between the back skin temperature at the time of sleeping of the sleeping person M for 240 min. when the human body cooling apparatus 301 pertaining to the present Embodiment 1 is used, and that when the same is not used, temperature measurement has been carried out at an ambient temperature of 30 deg. C.

As a result of this, it has been revealed that, when the human body cooling apparatus 301 is used, the skin temperature at the back of the sleeping person M from the start of sleeping until 240 min. has elapsed is held all the time to a skin temperature 2 to 3 deg. lower than that when the human body cooling apparatus 301 is not used.

As can be seen from the results of the temperature measurement as stated above, the human body cooling apparatus 301 pertaining to the present Embodiment 1 exhibits an excellent cooling effect during the sleep of the sleeping person M.

In addition, the human body cooling apparatus 301 pertaining to the present Embodiment 1 is also capable of reversible use by front/back reversing said exterior element 302, whereby the human body cooling apparatus 301 can withstand the use over a longer period of time.

### Embodiment 2:

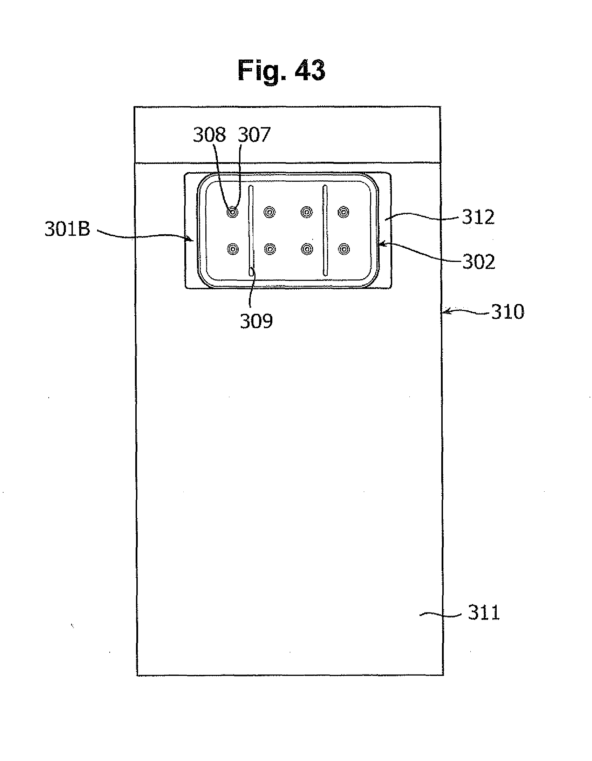

Next, with reference to FIG. 40 and FIG. 43, the mode of use, and the like, of the human body cooling apparatus 301A pertaining to the present Embodiment 2 will be described.

The human body cooling apparatus 301 A pertaining to the present Embodiment 2 has substantially the same configuration as that of the aforedescribed human body cooling apparatus 301, however, as shown in FIG. 40, it is configured to provide a half size, the entire size being reduced to that of, for example, a width of 90 cm, and a length of 90 cm, which is half the size of said human body cooling apparatus 301.

As shown in FIG. 40, said partially crimped portion 307 in this human body cooling apparatus 301 A is provided in twelve places in the lateral direction and ten places in the longitudinal direction over the entire area of the exterior element 302, in one hundred and twenty places in total. The air bleeder hole 308 is provided in each partially crimped portion 307 in the shape of a circle.

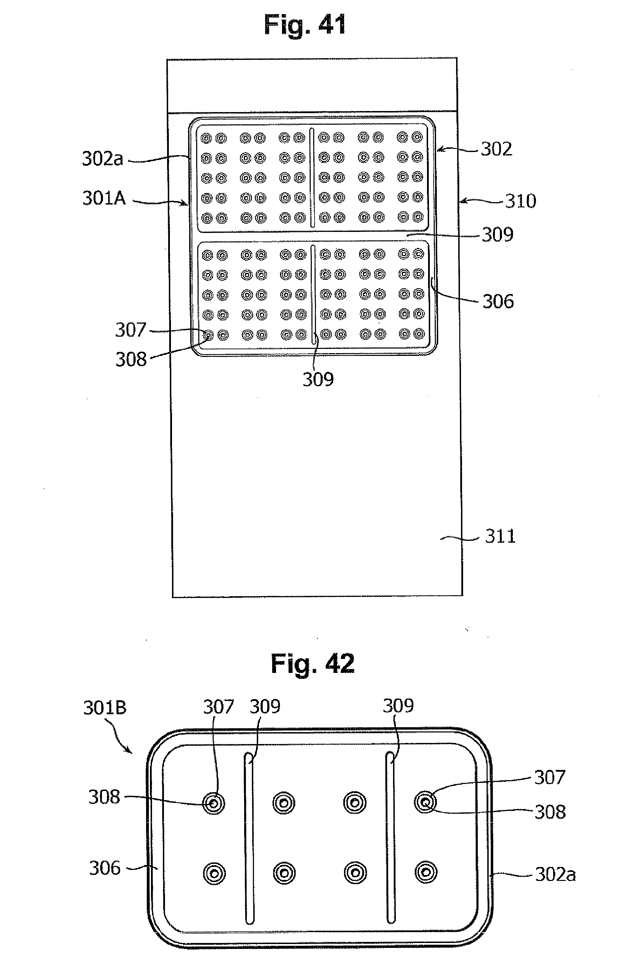

As shown in FIG. 41, the human body cooling apparatus 301A pertaining to the present Embodiment 2 is also used in such a mode as that in which it is placed on the top of a sheet 311 on a bed (or a futon mattress) 310 for use in sleeping, or the like, and a sleeping person M lies on said human body cooling apparatus 301A for sleep.

According to the human body cooling apparatus 301A pertaining to the present Embodiment 2, at the time of sleeping of the sleeping person M, the body heat at the back thereof, and the like, is taken away, whereby the cooling effect is enhanced, thus substantially the same functional effect as that with said human body cooling apparatus 301 can be obtained.

In addition, the human body cooling apparatus 301A pertaining to the present Embodiment 2 is also capable of reversible use by front/back reversing this human body cooling apparatus 301A, whereby the human body cooling apparatus 301A can withstand the use over a longer period of time.

### Embodiment 3:

A human body cooling apparatus 301B pertaining to the present Embodiment 3 will be described with reference to FIG. 42 and FIG. 43.

The human body cooling apparatus 301B pertaining to the present Embodiment 3 has substantially the same configuration as that of the aforedescribed human body cooling apparatus 301, however, as shown in FIG 42, it is configured for use as a sheet 312, the entire size being reduced to that of, for example, a width of 30 cm, and a length of 40 cm.

As shown in FIG. 42, with this human body cooling apparatus 301B, said partially crimped portion 307 is provided in four places in the lateral direction and two places in the longitudinal direction over the entire area of the exterior element 302, in eight places in total. The air bleeder hole 308 is provided in each partially crimped portion 307 in the shape of, for example, a circle.

FIG. 43 shows the mode of use of the human body cooling apparatus 301B pertaining to the present Embodiment 3. If the human body cooling apparatus 301B is put on the sheet 312, and the sleeping person M rests the head thereon for sleep, this human body cooling apparatus 301B is comfortably fitted to the head of the sleeping person M, in the same manner as is the case aforedescribed, and due to the temperature difference between the room temperature and the body temperature of the sleeping person M, the cooling medium 305 takes away the body heat of the head of the sleeping person M, with said air bleeder hole 308 dissipating the heat into the atmosphere in the room. In other words, radiation of the heat from the body of the sleeping person M is satisfactorily performed.

Thus, the human body cooling apparatus 301 pertaining to the present Embodiment 1 has excellent effects of giving a good feeling upon use to the sleeping person M, and moderately cooling the body temperature of the sleeping person M from the start of sleeping to awaking in the high temperature season while providing the state in which the head is kept cool, and the feet is kept warm, thus realizing comfortable sleep.

The aforementioned human body cooling apparatus 301, human body cooling apparatus 301A, and human body cooling apparatus 301B can each give a chill feeling, however, they can be used after being cooled in a refrigerator, or being warmed with a microwave oven.

### Industrial Applicability:

The first invention, the second invention, and the third invention are mainly used in sleeping as described above, and in addition, can be used for a wide variety of purposes, such as recovery from fatigue in the summer season, rest after sport, and the like.

## Claims

1. A human body cooling apparatus, comprising:
an exterior element in which two covering members in the shape of a rectangular seat each having a front cloth made of fabric, and a backing cloth which is weldable by high-frequency heating are put one upon another with said backing cloths being superposed on each other to provide a flat form; and
a cooling medium containing gel accommodated in said exterior element such that migration thereof is restricted by an outer edge crimped portion and a partially crimped portion in an internal area thereof that are produced by high-frequency welding of the backing cloths in said two covering members.

2. A human body cooling apparatus, comprising:
an exterior element in which two covering members in the shape of a rectangular seat each having a front cloth made of fabric, and a backing cloth which is weldable by high-frequency heating, of a size selected from various sizes such as, for example, a bed sheet size, a pillow size, a half size, i.e., a middle size between these sizes, further, a car seat size, a floor cushion size, a wine bottle heat insulating seat size, and the like, are put one upon another with said backing cloths being superposed on each other to provide a flat form; and
a cooling medium containing gel accommodated in said exterior element such that migration thereof is restricted by a four-side outer edge crimped portion and a partially crimped portion in an internal area thereof that are produced by high-frequency welding of the backing cloths in said two covering members.

3. A human body cooling apparatus, comprising:
an exterior element in which two covering members in the shape of a rectangular seat each having a front cloth made of fabric, and a backing cloth which is weldable by high-frequency heating, of a size selected from various sizes such as, for example, a bed sheet size, a pillow size, a half size, i.e., a middle size between these sizes, further, a car seat size, a floor cushion size, a wine bottle heat insulating seat size, and the like, are put one upon another with said backing cloths being superposed on each other to provide a flat form; and
a cooling medium containing gel accommodated in said exterior element such that migration thereof is restricted by a four-side outer edge crimped portion and a toric partially crimped portion in a plurality of places in an internal area thereof that are produced by high-frequency welding of the backing cloths in said two covering members.

4. A human body cooling apparatus, comprising:
an exterior element in which two covering members in the shape of a rectangular seat each having a front cloth made of fabric, and a backing cloth which is weldable by high-frequency heating, of a size selected from various sizes such as, for example, a bed sheet size, a pillow size, a half size, i.e., a middle size between these sizes, further, a car seat size, a floor cushion size, a wine bottle heat insulating seat size, and the like, are put one upon another with said backing cloths being superposed on each other to provide a flat form; and
a cooling medium containing gel accommodated in said exterior element such that migration thereof is restricted by a four-side outer edge crimped portion, a cross-shaped partially crimped portion connecting between the middle portions of the respective opposed sides in the four-side outer edge crimped portion, and a plurality of rectangular partially crimped portions in the respective four areas defined by the cross-shaped partially crimped portion that are produced by high-frequency welding of the backing cloths in said two covering members.

5. A human body cooling apparatus, comprising:
an exterior element in which two covering members in the shape of a rectangular seat each having a front cloth made of fabric, and a backing cloth which is weldable by high-frequency heating, of a size selected from various sizes such as, for example, a bed sheet size, a pillow size, a half size, i.e., a middle size between these sizes, further, a car seat size, a floor cushion size, a wine bottle heat insulating seat size, and the like, are put one upon another with said backing cloths being superposed on each other to provide a flat form; and
a cooling medium containing gel accommodated in said exterior element such that migration thereof is restricted by a four-side outer edge crimped portion and a plurality of corrugated partially crimped portions in an internal area thereof that are produced by high-frequency welding of the backing cloths in said two covering members.

6. A human body cooling apparatus, comprising:
an exterior element in which two covering members in the shape of a rectangular seat each having a front cloth made of fabric, and a backing cloth which is weldable by high-frequency heating, of a size selected from various sizes such as, for example, a bed sheet size, a pillow size, a half size, i.e., a middle size between these sizes, further, a car seat size, a floor cushion size, a wine bottle heat insulating seat size, and the like, are put one upon another with said backing cloths being superposed on each other to provide a flat form; and
a cooling medium containing gel accommodated in said exterior element such that migration thereof is restricted by a four-side outer edge crimped portion and a plurality of rectangular partially crimped portions in an internal area thereof that are produced by high-frequency welding of the backing cloths in said two covering members.

7. A human body cooling apparatus, comprising:
an exterior element in which two covering members in the shape of a rectangular seat each having a front cloth made of fabric, and a backing cloth which is weldable by high-frequency heating, of a size selected from various sizes such as, for example, a bed sheet size, a pillow size, a half size, i.e., a middle size between these sizes, further, a car seat size, a floor cushion size, a wine bottle heat insulating seat size, and the like, are put one upon another with said backing cloths being superposed on each other to provide a flat form; and
a cooling medium containing gel accommodated in said exterior element such that migration thereof is restricted by a four-side outer edge crimped portion and a plurality of partially crimped portions arranged in parallel to one another in an inclined disposition in an internal area thereof to exhibit a substantially rectangular space occupying configuration as a whole that are produced by high-frequency welding of the backing cloths in said two covering members.

8. A human body cooling apparatus, comprising:
an exterior element in which two covering members in the shape of a rectangular seat each having a front cloth made of fabric, and a backing cloth which is weldable by high-frequency heating, of a size selected from various sizes such as, for example, a bed sheet size, a pillow size, a half size, i.e., a middle size between these sizes, further, a car seat size, a floor cushion size, a wine bottle heat insulating seat size, and the like, are put one upon another with said backing cloths being superposed on each other to provide a flat form; and
a cooling medium containing gel accommodated in said exterior element such that migration thereof is restricted by a four-side outer edge crimped portion and a plurality of partially crimped portions arranged in a pattern of crosses in an internal area thereof, each cross exhibiting a substantially rectangular space occupying configuration as a whole, that are produced by high-frequency welding of the backing cloths in said two covering members.

9. A human body cooling apparatus, comprising:
an exterior element in which two covering members in the shape of a rectangular seat each having a front cloth formed of a material selected from cotton fiber, polyester, rayon, hemp, raised fabric, crepe, striped crepe, towel fabric, and the like, and a backing cloth formed of a non-water absorbent material, such as polyvinyl chloride, or the like, which is weldable by high-frequency heating are put one upon another with said backing cloths being superposed on each other to provide a flat form; and
a cooling medium containing gel, sodium polyacrylate, ammonium sulfate, sodium sulfate, glycerin, water, fungicide, and the like, accommodated in said exterior element such that migration thereof is restricted by an outer edge crimped portion and a partially crimped portion in an internal area thereof that are produced by high-frequency welding of the backing cloths in said two covering members.

10. A human body cooling apparatus, comprising:
an exterior element in which two covering members in the shape of a rectangular seat each having a front cloth formed of a material selected from cotton fiber, polyester, rayon, hemp, raised fabric, crepe, striped crepe, towel fabric, and the like, and a backing cloth formed of a non-water absorbent material, such as polyvinyl chloride, or the like, which is weldable by high-frequency heating, of a size selected from various sizes such as, for example, a bed sheet size, a pillow size, a half size, i.e., a middle size between these sizes, further, a car seat size, a floor cushion size, a wine bottle heat insulating seat size, and the like, are put one upon another with said backing cloths being superposed on each other to provide a flat form; and
a cooling medium containing gel, sodium polyacrylate, ammonium sulfate, sodium sulfate, glycerin, water, fungicide, and the like, accommodated in said exterior element such that migration thereof is restricted by a four-side outer edge crimped portion and a partially crimped portion in an internal area thereof that are produced by high-frequency welding of the backing cloths in said two covering members;
said exterior element being capable of reversible use by front/back reversing it.

11. A human body cooling apparatus, comprising:
an exterior element in which two covering members in the shape of a rectangular seat each having a front cloth formed of a material selected from cotton fiber, polyester, rayon, hemp, raised fabric, crepe, striped crepe, towel fabric, and the like, and a backing cloth formed of a non-water absorbent material, such as polyvinyl chloride, or the like, which is weldable by high-frequency heating, of a size selected from various sizes such as, for example, a bed sheet size, a pillow size, a half size, i.e., a middle size between these sizes, further, a car seat size, a floor cushion size, a wine bottle heat insulating seat size, and the like, are put one upon another with said backing cloths being superposed on each other to provide a flat form; and
a cooling medium containing gel, sodium polyacrylate, ammonium sulfate, sodium sulfate, glycerin, water, fungicide, and the like, accommodated in said exterior element such that migration thereof is restricted by a four-side outer edge crimped portion, and a toric partially crimped portion in a plurality of places in an internal area thereof that are produced by high-frequency welding of the backing cloths in said two covering members;
said exterior element being capable of reversible use by front/back reversing it.

12. A human body cooling apparatus, comprising:
an exterior element in which two covering members in the shape of a rectangular seat each having a front cloth formed of a material selected from cotton fiber, polyester, rayon, hemp, raised fabric, crepe, striped crepe, towel fabric, and the like, and a backing cloth formed of a non-water absorbent material, such as polyvinyl chloride, or the like, which is weldable by high-frequency heating, of a size selected from various sizes such as, for example, a bed sheet size, a pillow size, a half size, i.e., a middle size between these sizes, further, a car seat size, a floor cushion size, a wine bottle heat insulating seat size, and the like, are put one upon another with said backing cloths being superposed on each other to provide a flat form; and
a cooling medium containing gel, sodium polyacrylate, ammonium sulfate, sodium sulfate, glycerin, water, fungicide, and the like, accommodated in said exterior element such that migration thereof is restricted by a four-side outer edge crimped portion, a cross-shaped partially crimped portion connecting between the middle portions of the respective opposed sides in the four-side outer edge crimped portion, and a plurality of rectangular partially crimped portions in the respective four areas defined by the cross-shaped partially crimped portion that are produced by high-frequency welding of the backing cloths in said two covering members;
said exterior element being capable of reversible use by front/back reversing it.

13. A human body cooling apparatus, comprising:
an exterior element in which two covering members in the shape of a rectangular seat each having a front cloth formed of a material selected from cotton fiber, polyester, rayon, hemp, raised fabric, crepe, striped crepe, towel fabric, and the like, and a backing cloth formed of a non-water absorbent material, such as polyvinyl chloride, or the like, which is weldable by high-frequency heating, of a size selected from various sizes such as, for example, a bed sheet size, a pillow size, a half size, i.e., a middle size between these sizes, further, a car seat size, a floor cushion size, a wine bottle heat insulating seat size, and the like, are put one upon another with said backing cloths being superposed on each other to provide a flat form; and
a cooling medium containing gel, sodium polyacrylate, ammonium sulfate, sodium sulfate, glycerin, water, fungicide, and the like, accommodated in said exterior element such that migration thereof is restricted by a four-side outer edge crimped portion and a plurality of corrugated partially crimped portions in an internal area thereof that are produced by high-frequency welding of the backing cloths in said two covering members;
said exterior element being capable of reversible use by front/back reversing it.

14. A human body cooling apparatus, comprising:
an exterior element in which two covering members in the shape of a rectangular seat each having a front cloth formed of a material selected from cotton fiber, polyester, rayon, hemp, raised fabric, crepe, striped crepe, towel fabric, and the like, and a backing cloth formed of a non-water absorbent material, such as polyvinyl chloride, or the like, which is weldable by high-frequency heating, of a size selected from various sizes such as, for example, a bed sheet size, a pillow size, a half size, i.e., a middle size between these sizes, further, a car seat size, a floor cushion size, a wine bottle heat insulating seat size, and the like, are put one upon another with said backing cloths being superposed on each other to provide a flat form; and
a cooling medium containing gel, sodium polyacrylate, ammonium sulfate, sodium sulfate, glycerin, water, fungicide, and the like, accommodated in said exterior element such that migration thereof is restricted by a four-side outer edge crimped portion and a plurality of rectangular partially crimped portions in an internal area thereof that are produced by high-frequency welding of the backing cloths in said two covering members;
said exterior element being capable of reversible use by front/back reversing it.

15. A human body cooling apparatus, comprising:
an exterior element in which two covering members in the shape of a rectangular seat each having a front cloth formed of a material selected from cotton fiber, polyester, rayon, hemp, raised fabric, crepe, striped crepe, towel fabric, and the like, and a backing cloth formed of a non-water absorbent material, such as polyvinyl chloride, or the like, which is weldable by high-frequency heating, of a size selected from various sizes such as, for example, a bed sheet size, a pillow size, a half size, i.e., a middle size between these sizes, further, a car seat size, a floor cushion size, a wine bottle heat insulating seat size, and the like, are put one upon another with said backing cloths being superposed on each other to provide a flat form; and
a cooling medium containing gel, sodium polyacrylate, ammonium sulfate, sodium sulfate, glycerin, water, fungicide, and the like, accommodated in said exterior element such that migration thereof is restricted by a four-side outer edge crimped portion and a plurality of partially crimped portions arranged in parallel to one another in an inclined disposition in an internal area thereof to exhibit a substantially rectangular space occupying configuration as a whole that are produced by high-frequency welding of the backing cloths in said two covering members;
said exterior element being capable of reversible use by front/back reversing it.

16. human body cooling apparatus, comprising:
an exterior element in which two covering members in the shape of a rectangular seat each having a front cloth formed of a material selected from cotton fiber, polyester, rayon, hemp, raised fabric, crepe, striped crepe, towel fabric, and the like, and a backing cloth formed of a non-water absorbent material, such as polyvinyl chloride, or the like, which is weldable by high-frequency heating, of a size selected from various sizes such as, for example, a bed sheet size, a pillow size, a half size, i.e., a middle size between these sizes, further, a car seat size, a floor cushion size, a wine bottle heat insulating seat size, and the like, are put one upon another with said backing cloths being superposed on each other to provide a flat form; and
a cooling medium containing gel, sodium polyacrylate, ammonium sulfate, sodium sulfate, glycerin, water, fungicide, and the like, accommodated in said exterior element such that migration thereof is restricted by a four-side outer edge crimped portion and a plurality of partially crimped portions arranged in a pattern of crosses in an internal area thereof, each cross exhibiting a substantially rectangular space occupying configuration as a whole, that are produced by high-frequency welding of the backing cloths in said two covering members;
said exterior element being capable of reversible use by front/back reversing it.

17. A human body cooling apparatus, comprising:
an exterior element in which two covering members in the shape of a rectangular seat each having a front cloth formed of a fabric-like material, and a backing cloth formed of a non-water absorbent material which is weldable by high-frequency heating are put one upon another with said backing cloths being superposed on each other to provide a flat form, the backing cloths being high-frequency welded for formation of an outer edge crimped portion, a toric partially crimped portion having an air bleeder hole for heat radiation in a plurality of places in an internal area thereof, and straight-linear middle crimped portions; and
a cooling medium accommodated in said exterior element such that migration thereof is restricted by said outer edge crimped portion, partially crimped portions in the internal area thereof, and middle crimped portions.

18. A human body cooling apparatus, comprising:
an exterior element in which two covering members in the shape of a rectangular seat each having a front cloth formed of a material selected from cotton fiber, polyester, rayon, hemp, raised fabric, crepe, striped crepe, towel fabric, and the like, and a backing cloth formed of a non-water absorbent material, such as polyvinyl chloride, or the like, which is weldable by high-frequency heating are put one upon another with said backing cloths being superposed on each other to provide a flat form, the backing cloths being high-frequency welded for formation of an outer edge crimped portion, a toric partially crimped portion having an air bleeder hole for heat radiation in a plurality of places in an internal area thereof, and straight-linear middle crimped portions; and
a cooling medium containing gel, sodium polyacrylate, ammonium sulfate, sodium sulfate, glycerin, water, fungicide, and the like, accommodated in said exterior element such that migration thereof is restricted by said outer edge crimped portion, partially crimped portions in the internal area thereof, and middle crimped portions;
said exterior element being capable of reversible use by front/back reversing it.

19. A human body cooling apparatus, comprising:
an exterior element in which two covering members in the shape of a rectangular seat each having a front cloth formed of a material selected from cotton fiber, polyester, rayon, hemp, raised fabric, crepe, striped crepe, towel fabric, and the like, and a backing cloth formed of a non-water absorbent material, such as polyvinyl chloride, or the like, which is weldable by high-frequency heating, of a size selected from various sizes such as, for example, a bed sheet size, a pillow size, a half size, i.e., a middle size between these sizes, further, a car seat size, a floor cushion size, a wine bottle heat insulating seat size, and the like, are put one upon another with said backing cloths being superposed on each other to provide a flat form, the backing cloths being high-frequency welded for formation of an outer edge crimped portion, a toric partially crimped portion having an air bleeder hole for heat radiation in a plurality of places in an internal area thereof, and straight-linear middle crimped portions; and
a cooling medium containing gel, sodium polyacrylate, ammonium sulfate, sodium sulfate, glycerin, water, fungicide, and the like, accommodated in said exterior element such that migration thereof is restricted by said outer edge crimped portion, partially crimped portions in the internal area thereof, and middle crimped portions;
said exterior element being capable of reversible use by front/back reversing it.

20. A human body cooling apparatus, comprising:
an exterior element in which two covering members in the shape of a rectangular seat each having a front cloth formed of a material selected from cotton fiber, polyester, rayon, hemp, raised fabric, crepe, striped crepe, towel fabric, and the like, and a backing cloth formed of a non-water absorbent material, such as polyvinyl chloride, or the like, which is weldable by high-frequency heating, of a size selected from various sizes such as, for example, a bed sheet size, a pillow size, a half size, i.e., a middle size between these sizes, further, a car seat size, a floor cushion size, a wine bottle heat insulating seat size, and the like, are put one upon another with said backing cloths being superposed on each other to provide a flat form, the backing cloths being high-frequency welded for formation of an outer edge crimped portion, a partially crimped portion with a toric shape, square shape, triangular shape, oval shape, or the like, in a plurality of places in an internal area thereof that has an air bleeder hole for heat radiation with a shape selected from a circular shape, square shape, triangular shape, oval shape, and the like, and straight-linear middle crimped portions; and
a cooling medium containing gel, sodium polyacrylate, ammonium sulfate, sodium sulfate, glycerin, water, fungicide, and the like, accommodated in said exterior element such that migration thereof is restricted by said outer edge crimped portion, partially crimped portions in the internal area thereof, and middle crimped portions;
said exterior element being capable of reversible use by front/back reversing it.
